# EUROPEAN PATENT APPLICATION

(11) **EP 4 256 977 A1**
(43) Date of publication of application: **11.10.2023**
(21) Application number: 22166917.9
(22) Date of filing: 06.04.2022
(51) Int. Cl.: A23L 33/105, A23L 33/15, A23L 33/16, A61K 31/132, A61P 5/24, A61P 15/08

(54) **IMPROVING SEMEN QUALITY**

(71) Applicant: The Ceutics Company GesmbH, 1010 Vienna (AT)
(72) Inventor: FRITZ, Stefan Ulrich, 2371 Hinterbrühl (AT)
(74) Representative: Redl, Gerda

(57) **Abstract**

Composition for use in a method of improving the human male's semen quality, said composition comprising at least one source of spermidine, and optionally one or more further components and/or one or more carriers or excipients.

## Description

### FIELD OF THE INVENTION

The present invention relates to the field of food supplements and medical preparations for improving human male's semen quality and quantity, thereby contributing to male fertility.

### BACKGROUND OF THE INVENTION

Male fertility largely depends on the number and condition of the spermatozoa, as well as semen morphology and motility.

One of the most common causes of male infertility is inadequate sperm production, in particular caused by an inability to produce spermatozoa in sufficient quantities. This is called Azoospermia when there is no production of spermatozoa, whereas it is diagnosed as oligospermia when production of spermatozoa takes place, but production levels are very low.

Semen analysis is indicated for diagnosis of male infertility and to plan assisted fertilisation. Number of sperms in a single ejaculation, the volume of a single sperm and the motility of the sperms are a few factors that are taken into consideration during semen analysis. The seminogram test, also known as the spermiogram, aims to evaluate parameters for semen. These parameters are established on two levels, macroscopic and microscopic. The macroscopic parameters are: appearance, liquefaction, viscosity, pH, volume and colour. The microscopic parameters are: concentration and total number of spermatozoa, motility and vitality of sperm, sperm morphology and any presence of agglutination (antisperm antibodies). A laboratory manual for the examination and processing of human semen has been published by the World Health Organisation (Cooper, T. G. (ed.) WHO laboratory manual for the examination and processing of human semen, WHO Press, Geneva, Switzerland, 2010), establishing the guidelines for these and providing references and criteria for what is normal. This guide was updated in 2002 by the European Society of Human Reproduction and Embryology (ESHRE Monographs, Volume 2002, Issue 2, 15 June 2002, Manual on Basic Semen Analysis). These values are summarised in the present Examples.

AT503219B1 discloses vitamins and minerals in a combination preparation to improve semen quality.

Bauer et al. (Cell Cycle 2013; 12:2, 346-352) describe that spermidine, a naturally occurring polyamine, promotes mating and fertilization efficiency in model organisms., such as yeast or nematodes.

Lefevre et al. (Endocrine Reviews 2011, 32(5):694-712) review the role of polyamines, such as spermine, spermidine, and putrescine, on the reproductive landscape.

There is a need for new preparations and treatments to improve human male's semen quality.

### SUMMARY OF THE INVENTION

It is the objective of the present invention to provide a new method and preparation for administering to human male subjects for improving the semen quality.

The objective is solved by the subject of the claims and as further described herein.

Surprisingly, human male's semen quality can be improved upon oral intake of a preparation comprising a composition comprising a specific spermidine content, in particular spermidine from buckwheat sprout flour. The sperm quality was particular improved as determined in a seminogram, e.g., by increasing quantitative parameters such as the concentration and total number of spermatozoa, an/or by improving qualitative parameters such as sperm motility, sperm vitality, and sperm morphology, as determined by standard assays, such as according to ESHRE Monographs.

According to the invention there is provided a composition for use in a method of improving the human male's semen quality, said composition comprising at least one source of spermidine, and optionally one or more further components and/or one or more carriers or excipients.

Specifically, the at least one source of spermidine is comprised in an amount to provide a spermidine content of 3-8 mg, preferably 4-8 mg. According to a preferred aspect, the amount of spermidine administered is at least about any one of 3, 4, 5, 6, 7, or 8. Specifically preferred is an amount of about 6 mg.

According to a preferred aspect, the spermidine amount is provided in in one or more doses per day e.g., up to 5, 4, 3, 2, or 1 dose(s) per day.

Specifically, the spermidine dose (or in particular the daily dose) amounts to 3-8 mg, preferably 4-8 mg. According to a preferred aspect, the spermidine dose is at least about any one of 3, 4, 5, 6, 7, or 8. Specifically preferred is a dose of about 6 mg.

Specifically, the spermidine dose (or in particular the daily dose) amounts to 40-120 mg/kg body weight, preferably at least about any one of 50, 60, 70, 80, 90, 100, 110, or 120 mg/kg body weight, more preferably about 85 mg/kg body weight.

According to a specific aspect, the at least one source of spermidine is buckwheat sprout flour, preferably wherein the buckwheat sprout flour comprises 0.1 to 1% spermidine, preferably about 0.5%.

Specifically, the amount of buckwheat sprout flour is about 800 to 1600 mg, preferably about 1200 mg, or an amount of an alternative spermidine source, to provide a spermidine content of 3-8 mg, preferably 4-8 mg, preferably about 6 mg.

According to a specific aspect, the one or more further components are selected from one or more of the following:
a) plant extracts or powder, preferably one or more selected from; red ginseng root extract, maca root powder, tribulus terrestris fruit extract, ginko biloba leaf extract, tomato extract, chicory root extract;
b) at least one source of L-carnitine;
c) trace elements, preferably comprising one or both of zinc and selenium;
d) vitamins, preferably one or more selected from the group consisting of: folic acid, vitamin B6, vitamin B12, vitamin C, vitamin D3, vitamin E;
e) antioxidants, preferably comprising one or both of coenzyme Q₁₀ and N-acteyl-cysteine.

### Specifically:

a) the plant extracts or powder comprise:
   i. red ginseng root extract, preferably 1000 - 2000 mg, preferably about 1500 mg;
   ii. maca root powder, preferably 1000 - 2000 mg, preferably about 1650 mg;
   iii. tribulus terrestris fruit extract, preferably 50 - 150 mg, preferably about 90 mg; and/or an amount to provide a saponin content of 10 - 60 mg, preferably about 36 mg;
   iv. ginko biloba leaf extract, preferably 10 - 100 mg, preferably about 52 mg, and/or an amount to provide a flavonglycoside content of 5 - 20 mg, preferably about 12,5 mg; and
   v. tomato extract, preferably 100 - 200 mg, preferably about 150 mg, and/or an amount to provide a lycopene content of 10 - 20 mg, preferably about 15 mg;
   vi. chicory root extract, preferably 15 - 80 mg, preferably about 50 mg, and/or an amount to provide an inulin content of 10 - 60 mg, preferably about 40 mg;
b) the at least one source of L-carnitine is comprised in an amount to provide an L-carnitine content of 500 - 1000 mg, preferably about 680 mg, preferably wherein the source of L-carnitine is L-carnitine L-tartrate;
c) the trace elements comprise:
   i. zinc, preferably 10 - 50 mg, preferably about 28 mg, preferably in the form of zinc gluconate; and
   ii. selenium, preferably 10 - 100 µg, preferably about 55 µg, preferably in the form of sodium selenite or sodium selenite pentahydrate.
d) the vitamins comprise:
   i. folic acid, preferably 500 - 1000 µg, preferably about 800 µg, preferably in the form of pteroylmonoglutamic acid;
   ii. vitamin B6, preferably 1 - 6 mg, preferably about 3,5 mg, preferably in the form of pyridoxin hydrochloride, pyridoxal, or pyridoxamine;
   iii. vitamin B12, preferably 10 - 20 µg, preferably about 15 µg, preferably in the form of cyanocobalamin, or cobalamin;
   iv. vitamin C, preferably 100 - 500 mg, preferably about 250 mg, preferably in the form of L-ascorbic acid;
   v. vitamin D3, preferably 10 - 20 µg, preferably about 15 µg, preferably in the form of cholecalciferol, calciferol, calatriol or ergocalciferol; and
   vi. vitamin E, preferably 80 - 160 mg, preferably about 120 mg, preferably in the form of DL-α-Tocopherol acetate, alpha-Tocopherol, tocopherol or tocotrienol;
e) the antioxidants comprise:
   i. coenzyme Q10, preferably 50 - 150 mg, preferably about 100 mg; and
   ii. N-acteyl-cysteine, preferably 100 - 300 mg, preferably about 200 mg.

Specifically, the amount(s) of the component(s) of the composition described herein is/are understood as amount(s) administered as a dose per day (or daily dose/amounts). The composition may be provided in containments comprising one or more than one daily dose, such as e.g., in an amount to provide doses for a treatment period of at least 1 or more weeks, such as e.g., at least 1, 2, 3, 4, 5, 6, 7, or 8 weeks, or else a treatment period of at least 1 or more months, such as e.g., at least 1, 2, 3, 4, 5, or 6 months.

According to a specific aspect, the composition further comprises:
a) one or more dietary carrier substances, preferably comprising one or both of fiber and carbohydrates; and/or
b) one or more auxiliary agents, preferably selected from the group consisting of: sweeteners, food coloring agents, anti-caking agents, and flavoring agents.

Specific dietary carrier substances and/or auxiliary agents are selected from those as suitably used in a food product or food additive.

Preferred thickeners can be: guar gum or other natural thickeners.

Preferred sweeteners can be: stevia or other sweeteners.

Preferred food coloring agents can be carrot concentrate, black current concentrate and other fruit and vegetable-based concentrates.

A preferred release agent can be silicon dioxide.

Preferred flavoring agents can be: DL-malic acid, citric acid, and other natural aromas.

According to a specific aspect, the composition is used as a food supplement, food product, a nutraceutical product, dietetic and nutritional composition, a beverage, a medicament, a medicated food, a pharmaceutical composition, or a food for special medical purposes.

According to a specific aspect, the composition is in the form of a solid or liquid composition for oral use, in particular for oral intake use.

Specifically, the composition is comprised in an oral formulation. Specifically, the formulation is a preparation storage stable and/or ready-to-use.

According to a specific aspect, the composition is provided in the form of a powder, granulate, aqueous solution, suspension, capsule, tablet, or pellet.

According to a specific aspect, semen quality is improved as determined by a seminogram, in particular by one or more parameters a seminogram, including quantitative parameters such as sperm concentration, total sperm count, motility or viability, and/or qualitative parameters such as morphology. Preferably, one or more of: sperm concentration, total number of sperms, sperm motility, sperm vitality, and sperm morphology are improved.

Specifically, semen quality is improved as determined by at least one quantitative assay, preferably wherein improvement is at least any one of 1.1-fold, 1.2-fold, 1.3-fold, 1.4-fold, 1.5-fold, 1.6fold, 1.7-fold, 1.8-fold, 1.9-fold, or 2-fold.

The invention further provides for a food supplement or food supplement product, comprising
a) buckwheat sprout flour in an amount 800 to 1600 mg, preferably about 1200 mg, to provide a spermidine content of 3-8 mg, preferably 4-8 mg, preferably about 6 mg, or spermidine of alternative sources, to provide a spermidine content of 3-8 mg, preferably 4-8 mg, preferably about 6 mg;
b) plant extracts or powder, comprising:
   i. red ginseng root extract, preferably 1000 - 2000 mg, preferably about 1500 mg;
   ii. maca root powder, preferably 1000 - 2000 mg, preferably about 1650 mg;
   iii. tribulus terrestris fruit extract, preferably 50 - 150 mg, preferably about 90 mg; and/or to provide a saponin content of 10 - 60 mg, preferably about 36 mg;
   iv. ginko biloba leaf extract, preferably 10 - 100 mg, preferably about 52 mg, and/or to provide a flavonglycoside content of 5 - 20 mg, preferably about 12 mg; and
   v. tomatoe extract, preferably 100 - 200 mg, preferably about 150 mg, and/or to provide a lycopene content of 10 - 20 mg, preferably about 15 mg;
   vi. chicory root extract, preferably 15 - 80 mg, preferably about 50 mg, and/or an amount to provide an inulin content of 10 - 60 mg, preferably about 40 mg;
c) at least one source of L-carnitine, preferably 500 - 1000 mg L-carnitine, preferably about 680 mg, preferably wherein the source of L-carnitine is L-carnitine L-tartrate;
d) trace elements comprising:
   i. zinc, preferably 10 - 50 mg, preferably about 28 mg, preferably in the form of zinc gluconate; and
   ii. selenium, preferably 10 - 100 µg, preferably about 55 µg, preferably in the form of sodium selenite or sodium selenite pentahydrate;
e) vitamins comprising:
   i. folic acid, preferably 500 - 1000 µg, preferably about 800 µg, preferably in the form of pteroylmonoglutamic acid;
   ii. vitamin B6, preferably 1 - 6 mg, preferably about 3,5 mg, preferably in the form of pyridoxin hydrochloride, pyridoxal, or pyridoxamine;
   iii. vitamin B12, preferably 10 - 20 µg, preferably about 15 µg, preferably in the form of cyanocobalamin, or cobalamin;
   iv. vitamin C, preferably 100 - 500 mg, preferably about 250 mg, preferably in the form of L-ascorbic acid;
   v. vitamin D3, preferably 10 - 20 µg, preferably about 15 µg, preferably in the form of cholecalciferol, calciferol, calatriol or ergocalciferol; and
   vi. vitamin E, preferably 80 - 160 mg, preferably about 120 mg, preferably in the form of DL-α-Tocopherol acetate, alpha-Tocopherol, tocopherol or tocotrienol;
f) antioxidants comprising:
   i. coenzyme Q10, preferably 50 - 150 mg, preferably about 100 mg; and
   ii. N-acteyl-cysteine, preferably 100 - 300 mg, preferably about 200 mg.

Specifically, such food supplement is used in the treatment method as further described herein.

### DETAILED DESCRIPTION

Unless indicated or defined otherwise, all terms used herein have their usual meaning in the art, which will be clear to the skilled person. Specific terms as used throughout the specification have the following meaning.

Unless indicated otherwise, the term "about" as used herein refers to the same value or a value differing by +/-20 %, or +/-10 %, or +/-5 % of the given value. The term "about" as used for °C temperature typically refers to +/- 1°C.

To provide a more concise description, some of the quantitative expressions given herein are not qualified with the term "about". It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "seminogram" also termed "spermiogram" is understood in the following way. The spermiogram is a part of the andrological investigation, which includes physical, morphological as well as biochemical investigations of the ejaculate.

Semen quality is a measure of the ability of semen to accomplish fertilization. Thus, it is a measure of fertility in a male subject. Semen quality involves both sperm quantity and quality. Semen quality as described herein is specifically determined by a seminogram, and respective qualitative and quantitative parameters of semen analysis. Specifically, the parameters are one or more (or all of) of: sperm concentration, sperm count, motility, morphology, volume, fructose level and pH.

The term "sperm motility" as used herein refers to the ability of spermatozoa to move forward. In the present context, the term is to be understood as referring also to the motility grade, where the motility of sperm can be one of four different grades:
Grade a: Sperm with progressive motility. These are the strongest and swim fast in a straight line.
Grade b: (non-linear motility): These also move forward but tend to travel in a curved or crooked motion.
Grade c: These have non-progressive motility because they do not move forward despite the fact that they move their tails.
Grade d: These are immotile and fail to move at all.

The term "Total Motile Sperm Count" (TMSC) or "Total motile spermatozoa" (TMS), as used herein refers to a combination of sperm count, motility and volume, measuring how many million sperm cells in an entire ejaculate are motile.

A seminogram of a normal semen analysis typically comprises the results as shown in Table 1, or at least the respective results of: sperm concentration, total sperm count, motility and morphology.

**Table 1: World Health Organization Criteria for a Normal Semen Analysis**

| **Criteria** | **Parameters** |
|---|---|
| Volume | 2.0-5.0 mL |
| pH | 7.2-7.8 |
| Sperm concentration | ≥ 20 × 106/mL |
| Total sperm count | ≥ 40 × 106 spermatozoa |
| Motility | ≥ 50% with forward progression or ≥ 25% with rapid |
| | linear progression within 60 min after collection |
| Morphology | ≥ 50% with normal morphology |
| Viability | ≥ 75% live (ie, excluding dye) |
| White blood cells | ≤ 1 × 106/mL |
| Fructose (total) | ≥ 13 mol/ejaculate |

"Spermidine", as used herein, is a biogenic polyamine and a precursor to other polyamines, such as spermine. The term "spermidine" as used herein shall refer to any one or both of spermidine and/or salts of spermidine. Spermidine is present in most living organisms and plays a crucial role in growth. Spermidine as described herein can be used in the form of its pharmaceutically acceptable salts. The salts include, for example, salts with inorganic acids such as hydrochloric acid, sulfuric acid, phosphoric acid and boric acid, and salts with organic acids such as acetic acid, citric acid, tartaric acid and glutaric acid. Hence, the term "pharmaceutically acceptable salt" of spermidine refers to one or more of the aforementioned salts, but is not limited thereto and includes also other possible salts of spermidine.

Spermidine and/or pharmaceutically acceptable salts thereof can be obtained from several sources, such as of biogenic (including e.g., natural, or biotechnological sources) and/or synthetical origin. Plants and microorganisms (e.g., yeasts) represent a natural source of spermidine and pharmaceutically acceptable salts thereof. Specifically, a natural fruit, vegetable, legume, and/or grain-based food can be used as a source of spermidine. A preferred source is buckwheat sprout flour. It turned out that buckwheat sprout flour has a high concentration of spermidine. Suitable other preferred sources can be wheat sprout or soy bean, an extract of any of the foregoing. As an alternative to biogenic sources, synthetic spermidine pharmaceutically acceptable salts thereof can be obtained by suitable synthesis methods. Pharmaceutically acceptable spermidine salts can be obtained from biogenic or synthetic spermidine. Spermidine can also be produced by an enzymatic process, converting putrescine to spermidine e.g., using spermidine synthase.

Specifically, spermidine is provided in an extract of the source material, or in a solid source material that is e.g., powdered or granulated. A preferred source is buckwheat sprout flour (also referred to as buckwheat seedling flour, or buckwheat sprouted seed flour) or an extract thereof, in particular in the form of flour, which is provided as a solid powder or granulate.

It turned out that buckwheat sprout flour has a high concentration of spermidine. The spermidine concentration in buckwheat sprout flour is typically 0.1 to 1% (w/w) spermidine, preferably at least or about 0.5%.

A preferred source is organic sprouted buckwheat flour, such as commercially available Buckmidine^{®} (The Ceutics Company GesmbH, Austria).

Spermidine and/or pharmaceutically acceptable salts thereof of biotechnological origin, can be obtained also through other production methods such as well-known expression systems via recombinant expression of the enzymes involved in the synthesis of spermidine, including spermidine synthase (converting putrescine to spermidine). These would also be suitable as preferred sources.

Studies have determined that a daily intake of 12 mg of spermidine provided in a plant extract (e.g., wheat germ extract) is safe (Food and Drug Administration GRAS Notice No. 889 Spermidine-Rich Wheat Germ Extract). Such level of spermidine intake is far below the NOAEL (No Observed Adverse Effect Level) of 83 mg/kg body weight/day for spermidine (470 mg/day for a 70 kg person) (Til et al., Food and Chemical Toxicology 1997, v. 35, p. 337-348).

As used herein, the term "plant extract" refers to a substance that has been removed from plant material. Plant extracts may be prepared by treating any plant material with a suitable extractant to obtain an extract comprising at least some components of the plant material from the plant material in aqueous solution or suspension. The plant material which is treated with extractant to provide a plant extract may be or may be derived from any part of a plant, for example, the leaves, bark, roots, flowers, seeds or fruits of the plant, preferably it is the leaves, stem or flower of the plant or the peel of the fruit or any combination thereof.

Methods for the preparation of plant extracts are well known to the person skilled in the art. Specifically, the plant extracts can be obtained by decoction, digestion, percolation, soxlethtation, maceration or any other appropriate extraction method known to the person skilled in the art. Specifically, the plant extracts described herein are extracted using water or an aqueous solvent, specifically comprising or consisting of hot or cold water.

In the preparation of plant extract, the plant components extracted from the plant material will be at least partially soluble or suspendible in the extractant. After the plant material has been treated with the extractant, the insoluble plant material may be left in the extractant, or the insoluble plant material may be separated from the extractant. The insoluble plant material may be separated from the extractant according to any appropriate method, for example, decantation, filtration or centrifugation.

Specifically, the insoluble plant material is separated from the extractant, and the plant extract does not comprise insoluble components.

Specifically, the plant extract is an extractant-free extract. That is, a plant extract is preferably used where the extractant has been sufficiently removed, such as to provide a dry or non-aqueous extract.

As used herein, the term "plant powder" refers to a substance that is obtainable or obtained from plant material by grinding and drying. Plant powder includes any kind of powdered, or granulated plant material. The plant material which is treated to provide a plant powder may be or may be derived from any part of a plant, for example, the leaves, bark, roots, flowers, seeds or fruits of the plant, preferably it is the leaves, stem or flower of the plant or the peel of the fruit or any combination thereof.

As used herein, the term "subject" or "individual" shall refer to a warm-blooded mammalian, particularly a human being e.g., an adult. The subject may be a patient, specifically a patient in need of treatment, such as prophylaxis or therapy of a disease or disorder described herein.

The term "patient" includes human subjects that receive either prophylactic or therapeutic treatment or are at risk of or diagnosed of a disease or disorder that results in low semen quality, like, medication-induced, environment-caused or age-related abnormalities, or abnormalities affecting organs or the hormone system. The respective disorder or disease can be chronic or acute. Semen quality improvement can also be indicated where the subject comprises one or more of the risk factors of male infertility, such as smoking tobacco, using (or abusing) alcohol, using certain illicit drugs, being overweight, having certain past or present infections, being exposed to toxins, overheating the testicles, having experienced trauma to the testicles, or where likelihood of ovary fertilization (e.g., IVF) shall be increased. Specifically, a patient is treated as described herein, who suffers from male infertility, and/or low semen quality e.g., characterized by a low amount of one or more of sperm concentration, sperm count, or sperm motility, such as less than the normal value e.g., less than the medium or lower end of the normal range, or even less than any one of 90%, 80%, 70%, 60%, 50%, 40%, 30%, 20%, or 10% of the less than the medium or lower end of the normal range.

The term "treatment" is meant to include both prophylactic and therapeutic treatment.

According to a specific aspect described herein, spermidine is administered in an effective amount or therapeutically effective amount.

The term "effective amount" with respect to the effect on semen quality as described herein, shall refer to the quantity or activity sufficient to, when administered to a subject, effect beneficial of desired results, and, as such, an effective amount or synonym thereof depends upon the context in which it is being applied. In particular, an effective amount is understood to be a predetermined amount that has a proven effect to improve semen quality e.g., to normalize a seminogram, such as to obtain normal values of a seminogram.

Specifically, the effective amount of the composition described herein is determined by the effective amount of spermidine comprised in the composition.

The term "therapeutically effective amount", as used herein, means that amount of a compound or combination that will elicit the desired biological or medical response in a human male subject.

A treatment regime of a subject with an effective amount of the composition described herein may consist of a single application or administration, or alternatively comprise a series of applications and administrations, respectively. For example, the composition may be used at least once a week, or at least once a day. However, in certain cases, the composition may be used more frequently e.g., 1-5 times a day. The composition described herein may be provided for single or multiple dosage use.

The length of the treatment period depends on a variety of factors, such as the severity of the disease or disorder, either acute or chronic disease, the age of the patient, and the concentration of the spermidine in the composition. It will also be appreciated that the effective dosage used for the treatment may increase or decrease over the course of a particular treatment regime. Treatment results and changes in dosage may result and become apparent by standard diagnostic assays known in the art.

Unit-dose or multi-dose containers may be used, for example, sealed containers, and may be stored in a storage stable package. The composition may be provided as a dry formulation composition or comprising a liquid phase. Exemplary compositions may be provided as a dry e.g., freeze-dried (lyophilized), composition requiring only the addition of a liquid, such as water, immediately prior to use. Preferred unit dosage formulations are those containing a daily dose or unit daily sub-dose, or multiple doses, composition.

The composition described herein can be administered to an individual by various routes. The formulation and dosage forms used to administer the composition are preferably adapted to the route of administration. A preferred formulation is an oral formulation. A preferred administration route is the oral administration.

As used herein, the term "oral formulation" refers to any composition or pharmaceutically acceptable formulation, which is intended for peroral use, in particular for administration into the mouth of a subject.

Specifically, the composition is formulated in a solid dosage form such as a capsule, granulate, powder, or tablet, or liquid dosage forms such as a suspension, solution, a gel.

The composition described herein may comprise various carrier and/or excipients depending on the route of administration. The use of carriers and/or excipients for the type of compositions described herein or pharmaceutically active substances is well known in the art. Exemplary pharmaceutically acceptable carriers can be auxiliary substances and additives as further described herein.

Tablets of the present invention may be made by compression or molding, optionally with one or more additional ingredients. Compressed tablets may be prepared using a binder (e.g., gelatin or hydroxypropylmethyl cellulose), a lubricant, an inert diluent, a preservative, a disintegrant (e.g., sodium starch glycolate or cross-linked sodium carboxymethyl cellulose), and/or a surface-active or a dispersing agent.

The solid dosage form of the compositions can be prepared with coatings, such as enteric coatings and other coatings well known in the art. They may also be formulated so as to provide slow or controlled release of the active ingredient therein using e.g., hydroxypropylmethyl cellulose in varying proportions to provide the desired release profile, other polymer matrices, liposomes or microspheres.

Liquid dosage forms for oral administration of the composition may include pharmaceutically acceptable emulsions, microemulsions, solutions, suspensions, syrups and elixirs. The liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3- butylene glycol, oils (e.g., cottonseed, groundnut, corn, germ, olive, castor and sesame oils), glycerol, tetrahydrofuryl alcohol, polyethylene glycols and fatty acid esters of sorbitol, and mixtures thereof. Besides inert diluents, the oral compositions may also include adjuvants such as wetting agents, emulsifying and suspending agents, sweetening, flavoring, coloring, perfuming and preservative agents. Suspensions may contain suspending agents as, for example, ethoxylated isostearyl alcohols, polyoxyethylene sorbitol and sorbitan esters, microcrystalline cellulose, aluminum metahydroxide, bentonite, agar-agar and mixtures thereof.

The composition described herein may comprise one or more carriers and/or excipient that are pharmaceutically acceptable. The composition can be formulated with conventional carriers and/or excipients, which will be selected in accordance with ordinary practice.

The preferred composition is in a storage stable form, with a shelf-life of at least one or two years.

One or more carriers may be used appropriate a desired route of administration. The composition may be *e.g.,* comprise one or more edible carriers, such as carbohydrates like lactose, sucrose, or starch, or may comprise cellulose esters of alkanoic acids, stearic acid, talc, magnesium stearate, magnesium oxide, sodium and calcium salts of phosphoric and sulphuric acids, acacia, gelatin, sodium alginate, polyvinylpyrrolidine, polyvinyl alcohol, and optionally further tableted or encapsulated for conventional administration. Tablets may contain excipients, glidants, fillers, binders, disintegrants, lubricants, flavors and the like. Granules may be produced using isomaltose.

The composition may be dispersed or dissolved in saline, water, polyethylene glycol, propylene glycol, carboxymethyl cellulose colloidal solutions, ethanol, corn oil, peanut oil, cotton seed oil, sesame oil, tragacanth gum, and/or various buffers. Liquid formulations can be solutions, emulsions or suspensions and can include excipients such as suspending agents, solubilizers, surfactants, preservatives, and chelating agents. Typical carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, tragacanth, and sodium alginate; typical wetting agents include lecithin and polysorbate 80; and typical preservatives include methyl paraben and sodium benzoate.

Oral compositions may also contain one or more components such as sweeteners, flavoring agents and colorants as well-known in the art.

A carrier may also include a controlled release material or time delay material, such as glyceryl monostearate or glyceryl distearate alone or with a wax, or other materials, as well-known in the art.

Compositions may also be coated by conventional methods, typically with pH or time-dependent coatings, such that the subject agent is released in the gastrointestinal tract in the vicinity of the desired topical application, or at various times to extend the desired action. Such dosage forms typically include, but are not limited to, one or more of cellulose acetate phthalate, polyvinylacetate phthalate, hydroxypropyl methyl cellulose phthalate, ethyl cellulose, waxes, and shellac.

Other carriers, auxiliaries, and modes of administration are well known in the pharmaceutical arts.

The term "pharmaceutically acceptable" refers to an ingredient in a composition or formulation for medicinal or medical use, other than an active ingredient, without undue toxicity, incompatibility, instability, irritation, allergic response, and the like. A pharmaceutically acceptable carrier includes, but is not limited to, a buffer, excipient, stabilizer, or preservative, and in particular saline, phosphate buffered saline, dextrose, glycerol, ethanol, and the like.

The composition provided herein can be produced or manufactured by conventional processes known to the person skilled in the art. Specifically, the composition provided herein is produced by incorporation of the components of the composition described herein in a pharmaceutically acceptable formulation comprising pharmaceutically acceptable carriers. Specifically, the composition is produced by formulation of the composition described herein with pharmaceutically acceptable carriers, preferably in such a way that it can be applied as an oral formulation.

The examples described herein are illustrative of the present invention and are not intended to be limitations thereon. Many modifications and variations may be made to the techniques described and illustrated herein without departing from the spirit and scope of the invention. Accordingly, it should be understood that the examples are illustrative only and are not limiting upon the scope of the invention.

### EXAMPLES

### Example 1: Manufacturing a spermidine composition

The following ingredients were used as dry components of a powder mix called "Men Fertility Powder", prepared for a daily dose of 8g by oral intake.

Ingredients: maca root powder, red ginseng root extract, buckwheat sprout flour (including spermidine), Lcarnitine-L- tartrate, DLalpha-tocopheryl acetate, natural flavouring, L-ascorbic acid, acid: DL-malic acid, zinc gluconate, N-acetyl-L-cysteine,tomato extract (10% lycopene), natural flavouring, natural flavouring, coenzyme Q10, Tribulus terrestris fruit extract (40% saponins), thickener: guar gum, acid: citric acid, natural flavouring, ginkgo biloba leaf extract (24% flavonglycosides), inulin, (chicory), anti-caking agent: silicone dioxide, colouring food (carrot concentrate, black currant concentrate), sweetener: steviol glycosides from stevia, cyanocobalamin, sodium selenite, cholecalciferol, pyridoxine hydrochloride, pteroylmonoglutamic acid.

The composition is characterized as follows:

| | Per daily serving (8g) |
|---|---|
| Vitamin B6 | 3,5 mg |
| Folic acid | 800 µg |
| Vitamin B12 | 15 µg |
| Vitamin C | 250 mg |
| Vitamin D | 15 µg |
| Vitamin E | 120 mg |
| Selenium | 55,3 µg |
| Zinc | 27,7 mg |
| Maca root powder | 1650mg |
| Coenzyme Q10 | 100 mg |
| Gingko biloba leaf extract, thereof Flavonglykosides | 52,1 mg |
| | 12,5 mg |
| Tomato extract, thereof Lycopene | 150 mg |
| | 15 mg |
| Inulin | 40 mg |
| L-Carnitine | 680 mg |
| N-Acteyl-L-Cysteine | 200 mg |
| Red Ginseng root extract | 1500 mg |
| Tribulus terrestris fruit extract, thereof Saponins | 90 mg 36 mg |
| Buckwheat seedling flour, thereof spermidine | 1200 mg |
| | 6 mg |

### Example 2: Clinical test

Clinical Test: A 3-months study of 10 subjects. The trial assesses the positive effect of the composition on sperm quality, as well as safety linked to spermidine supplementation.

The purpose of this study is to observe the effect and side effects of the Men Fertility Powder produced according to Example 1, on sperm quality.

This observational study is conducted to analyse how sperm quality improves during a 90-day period whilst taking a new dietary supplement.

The study takes 3 months. At the start of the study, a seminogram, a blood test is done and the blood pressure is measured. During the study, the Men Fertility Powder is used daily, one serving per day, for 90 days.

8g are suspended in an adequate amount of cold water (or juice) and mixed to prepare a drink. Following the 90 days treatment period, another seminogram and blood test is made as well as a measurement of blood pressure.

The blood tests include the analysis of testosterone, prolactin and FSH. All three are key hormones related to sperm quality and male fertility.

The results of the study are provided in a respective seminogram, determined by standard assays, according to ESHRE Monographs.

### Seminogram:

| **General values** | | **Normal values of the WHO**** |
|---|---|---|
| Volume of sample ** | | 1.5 milliliters and more |
| liquefaction time | | Within 30-60 minutes |
| pH - value | | 7.2 to 8.0 |
| **leukocytes** | | **Less than 1 million / ml** |
| | | |

| **Special values** | | |
|---|---|---|
| Concentrated / dense sperm per milliliter (ml) | | more than 20/15 ** million sperm per milliliter (ml) |
| Mobility of spermatozoa in%: | | at least 40% ** |
| | a. fast moving forward | more than 25% |
| | b. total forward | a + b more than 32% ** (50%) |
| | c. local Mobile | less than 50% |
| | d. motionless | d less than 50% |
| | agility | a + b + c ** at least 40% |
| Morphology / shape of the sperm cells in% | | Unobtrusively shaped spermatozoa> 4% ** (30%) |
| ** WHO 5th Ed. | | |

## Claims

1. Composition for use in a method of improving the human male's semen quality, said composition comprising at least one source of spermidine, and optionally one or more further components and/or one or more carriers or excipients.

2. Composition for use according to claim 1, wherein the at least one source of spermidine is comprised in an amount to provide a spermidine content of 3-8 mg, preferably about 6 mg.

3. Composition for use according to claim 1 or 2, wherein the at least one source of spermidine is buckwheat sprout flour, preferably wherein the buckwheat sprout flour comprises 0.1 to 1% spermidine, preferably about 0.5%.

4. Composition for use according to any one of claims 1 to 3, wherein the one or more further components are selected from one or more of the following:
a) plant extracts or powder, preferably one or more selected from; red ginseng root extract, maca root powder, tribulus terrestris fruit extract, ginko biloba leaf extract, tomato extract, chicory root extract;
b) at least one source of L-carnitine;
c) trace elements, preferably comprising one or both of zinc and selenium;
d) vitamins, preferably one or more selected from the group consisting of: folic acid, vitamin B6, vitamin B12, vitamin C, vitamin D3, vitamin E;
e) antioxidants, preferably comprising one or both of coenzyme Q₁₀ and N-acteyl-cysteine.

5. Composition for use according to claim 4, wherein:
a) the plant extracts or powder comprise:
i. red ginseng root extract, preferably 1000 - 2000 mg, preferably about 1500 mg;
ii. maca root powder, preferably 1000 - 2000 mg, preferably about 1650 mg;
iii. tribulus terrestris fruit extract, preferably 50 - 150 mg, preferably about 90 mg; and/or an amount to provide a saponin content of 10 - 60 mg, preferably about 36 mg;
iv. ginko biloba leaf extract, preferably 10 - 100 mg, preferably about 52 mg, and/or an amount to provide a flavonglycoside content of 5 - 20 mg, preferably about 12,5 mg; and
v. tomato extract, preferably 100 - 200 mg, preferably about 150 mg, and/or an amount to provide a lycopene content of 10 - 20 mg, preferably about 15 mg;
vi. chicory root extract, preferably 15 - 80 mg, preferably about 50 mg, and/or an amount to provide an inulin content of 10 - 60 mg, preferably about 40 mg;
b) the at least one source of L-carnitine is comprised in an amount to provide an L-carnitine content of 500 - 1000 mg, preferably about 680 mg, preferably wherein the source of L-carnitine is L-carnitine L-tartrate;
c) the trace elements comprise:
i. zinc, preferably 10 - 50 mg, preferably about 28 mg, preferably in the form of zinc gluconate; and
ii. selenium, preferably 10 - 100 µg, preferably about 55 µg, preferably in the form of sodium selenite or sodium selenite pentahydrate.
d) the vitamins comprise:
i. folic acid, preferably 500 - 1000 µg, preferably about 800 µg, preferably in the form of pteroylmonoglutamic acid;
ii. vitamin B6, preferably 1 - 6 mg, preferably about 3,5 mg, preferably in the form of pyridoxin hydrochloride, pyridoxal, or pyridoxamine;
iii. vitamin B12, preferably 10 - 20 µg, preferably about 15 µg, preferably in the form of cyanocobalamin, or cobalamin;
iv. vitamin C, preferably 100 - 500 mg, preferably about 250 mg, preferably in the form of L-ascorbic acid;
v. vitamin D3, preferably 10 - 20 µg, preferably about 15 µg, preferably in the form of cholecalciferol, calciferol, calatriol or ergocalciferol; and
vi. vitamin E, preferably 80 - 160 mg, preferably about 120 mg, preferably in the form of DL-α-Tocopherol acetate, alpha-Tocopherol, tocopherol or tocotrienol;
e) the antioxidants comprise:
i. coenzyme Q10, preferably 50 - 150 mg, preferably about 100 mg; and
ii. N-acteyl-cysteine, preferably 100 - 300 mg, preferably about 200 mg.

6. Composition for use according to any one of claims 1 to 5, wherein the composition further comprises:
a) one or more dietary carrier substances, preferably comprising one or both of fiber and carbohydrates; and/or
b) one or more auxiliary agents, preferably selected from the group consisting of: sweeteners, food coloring agents, anti-caking agents, and flavoring agents.

7. Composition for use according to any one of claims 1 to 6, wherein the composition is used as a food supplement, food product, a nutraceutical product, dietetic and nutritional composition, a beverage, a medicament, a medicated food, a pharmaceutical composition, or a food for special medical purposes.

8. Composition for use according to any one of claims 1 to 7, wherein the composition is in the form of a solid or liquid composition for oral use.

9. Composition for use according to any one of claims 1 to 8, wherein the composition is provided in the form of a powder, granulate, aqueous solution, suspension, capsule, tablet, or pellet.

10. Composition for use according to any one of claims 1 to 9, wherein semen quality is improved as determined by a seminogram, preferably by improving one or more of: sperm concentration, total number of sperms, sperm motility, sperm vitality, and sperm morphology.

11. Composition for use according to claim 10, wherein the semen quality is improved as determined by a quantitative assay, preferably wherein improvement is at least 1.1-fold.

12. Food supplement product comprising
a) buckwheat sprout flour in an amount of 800 to 1600 mg, preferably about 1200 mg, to provide a spermidine content of 3-8 mg, preferably 4-8 mg, preferably about 6 mg, or spermidine of alternative sources, to provide a spermidine content of 3-8 mg, preferably 4-8 mg, preferably about 6 mg;
b) plant extracts or powder, comprising:
i. red ginseng root extract, preferably 1000 - 2000 mg, preferably about 1500 mg;
ii. maca root powder, preferably 1000 - 2000 mg, preferably about 1650 mg;
iii. tribulus terrestris fruit extract, preferably 50 - 150 mg, preferably about 90 mg; and/or to provide a saponin content of 10 - 60 mg, preferably about 36 mg;
iv. ginko biloba leaf extract, preferably 10 - 100 mg, preferably about 52 mg, and/or to provide a flavonglycoside content of 5 - 20 mg, preferably about 12 mg; and
v. tomatoe extract, preferably 100 - 200 mg, preferably about 150 mg, and/or to provide a lycopene content of 10 - 20 mg, preferably about 15 mg;
vi. chicory root extract, preferably 15 - 80 mg, preferably about 50 mg, and/or an amount to provide an inulin content of 10 - 60 mg, preferably about 40 mg;
c) at least one source of L-carnitine, preferably 500 - 1000 mg L-carnitine, preferably about 680 mg, preferably wherein the source of L-carnitine is L-carnitine L-tartrate;
d) trace elements comprising:
i. zinc, preferably 10 - 50 mg, preferably about 28 mg, preferably in the form of zinc gluconate; and
ii. selenium, preferably 10 - 100 µg, preferably about 55 µg, preferably in the form of sodium selenite or sodium selenite pentahydrate;
e) vitamins comprising:
i. folic acid, preferably 500 - 1000 µg, preferably about 800 µg, preferably in the form of pteroylmonoglutamic acid;
ii. vitamin B6, preferably 1 - 6 mg, preferably about 3,5 mg, preferably in the form of pyridoxin hydrochloride, pyridoxal, or pyridoxamine;
iii. vitamin B12, preferably 10 - 20 µg, preferably about 15 µg, preferably in the form of cyanocobalamin, or cobalamin;
iv. vitamin C, preferably 100 - 500 mg, preferably about 250 mg, preferably in the form of L-ascorbic acid;
v. vitamin D3, preferably 10 - 20 µg, preferably about 15 µg, preferably in the form of cholecalciferol, calciferol, calatriol or ergocalciferol; and
vi. vitamin E, preferably 80 - 160 mg, preferably about 120 mg, preferably in the form of DL-α-Tocopherol acetate, alpha-Tocopherol, tocopherol or tocotrienol;
f) antioxidants comprising:
i. coenzyme Q10, preferably 50 - 150 mg, preferably about 100 mg; and
ii. N-acteyl-cysteine, preferably 100 - 300 mg, preferably about 200 mg.
